# EUROPEAN PATENT APPLICATION

(11) **EP 4 717 182 A1**
(43) Date of publication of application: **01.04.2026**
(21) Application number: 25197475.4
(22) Date of filing: 21.08.2025
(51) Int. Cl.: A61B 8/00

(54) **ULTRASOUND PROBE**

(30) Priority: 30.09.2024 JP 2024170047
(71) Applicant: FUJIFILM Corporation, Tokyo 106-8620 (JP)
(72) Inventor: NOGUCHI, Shinsuke, Kaisei (JP); TSUBOTA, Keiji, Kaisei (JP)
(74) Representative: Meissner Bolte Partnerschaft mbB

(57) **Abstract**

Provided is an ultrasound probe that is easy to grip and can be reduced in size while a battery is built therein.

An ultrasound probe includes a housing that has a front end portion and a rear end portion, a transducer array that is disposed inside the front end portion of the housing, and a battery that has a flat plate shape and that is disposed inside the housing, in which the housing has an outer shape that gradually tapers along a center line extending from the front end portion to the rear end portion, from a vicinity of a center portion of the center line toward the front end portion or the rear end portion, and the battery is disposed inclined relative to the center line so as to lie along an inner surface of the housing.

## Description

### BACKGROUND OF THE INVENTION

### 1. Field of the Invention

The present invention relates to an ultrasound probe, and particularly to a wireless-connection ultrasound probe provided with a built-in battery.

### 2. Description of the Related Art

In related art, an ultrasound diagnostic apparatus using ultrasound images is put into practical use in the medical field. In general, such an ultrasound diagnostic apparatus comprises an ultrasound probe in which a transducer array is built and an apparatus body connected to the ultrasound probe, in which an ultrasound image is generated by transmitting an ultrasound beam from the ultrasound probe toward a subject, receiving an ultrasound echo from the subject by the ultrasound probe, and electrically processing the received signal, for example, in the apparatus body.

In recent years, as disclosed in JP2005-517515A and JP2016-105745A, an ultrasound diagnostic apparatus in which a battery is built in an ultrasound probe, and the ultrasound probe and an apparatus body are wirelessly connected to each other by wireless communication has been developed.

In such an ultrasound diagnostic apparatus, a cable for connecting the ultrasound probe and the apparatus body is not required, so that the operability and mobility of the ultrasound probe by a user can be improved.

### SUMMARY OF THE INVENTION

Since the ultrasound probe is used while performing a scan along a body surface of the subject in a state in which a user such as a doctor or a technician grips the ultrasound probe with one hand, the ultrasound probe is required to have a shape that is easy to grip and to be compact in size.

However, in a case in which the battery is to be built in the ultrasound probe, there is a problem that it is difficult to reduce the size of the ultrasound probe because it is necessary to ensure an accommodation space for the battery in a housing of the ultrasound probe, and it is desired to incorporate a circuit that drives a transducer array and that processes a received signal acquired by the transducer array, a wireless communication circuit that wirelessly transmits a signal from the ultrasound probe to the apparatus body, and the like in the ultrasound probe.

The present invention has been made in order to solve such a problem in the related art, and an object of the present invention is to provide an ultrasound probe that is easy to grip and can be reduced in size while a battery is built therein.

The above object can be achieved with the following configurations.
[1] An ultrasound probe with a built-in battery, the ultrasound probe comprising: a housing that has a front end portion and a rear end portion; a transducer array that is disposed inside the front end portion of the housing; and a battery that has a flat plate shape and that is disposed inside the housing, in which the housing has an outer shape that gradually tapers along a center line extending from the front end portion to the rear end portion, from a vicinity of a center portion of the center line toward the front end portion or the rear end portion, and the battery is disposed inclined relative to the center line so as to lie along an inner surface of the housing.
[2] The ultrasound probe according to [1], in which the housing has an outer shape that gradually tapers from the vicinity of the center portion of the center line toward the front end portion, and the battery is inclined relative to the center line at a position offset toward a side of the front end portion inside the housing.
[3] The ultrasound probe according to [2], further comprising: a power receive coil that is disposed inside the housing, in which the transducer array, the battery, and the power receive coil are disposed in this order from the front end portion of the housing toward the rear end portion of the housing.
[4] The ultrasound probe according to [3], in which the center line passes through the transducer array, and the power receive coil is disposed parallel to the center line at a position offset from the center line.
[5] The ultrasound probe according to [4], further comprising: a circuit board that is disposed inside the housing at a position offset with respect to the center line toward a side opposite to the battery and the power receive coil; and an integrated circuit that is mounted on the circuit board.
[6] The ultrasound probe according to [5], further comprising: a wireless communication circuit that is mounted on the circuit board.
[7] The ultrasound probe according to [1], in which the housing has an outer shape that gradually tapers from the vicinity of the center portion of the center line toward the rear end portion, and the battery is inclined relative to the center line at a position offset toward a side of the rear end portion inside the housing.

The ultrasound probe comprises: the housing that has the front end portion and the rear end portion; and the battery that has the flat plate shape and that is disposed inside the housing, the housing has the outer shape that gradually tapers along the center line extending from the front end portion to the rear end portion, from the vicinity of the center portion of the center line toward the front end portion or the rear end portion, and the battery is disposed inclined relative to the center line so as to lie along the inner surface of the housing, so that it is possible to provide the ultrasound probe that is easy to grip and can be reduced in size while the battery is built therein.

### BRIEF DESCRIPTION OF THE DRAWINGS

FIG. 1 is a perspective view showing an ultrasound probe according to a first embodiment of the present invention.
FIG. 2 is a plan view showing the ultrasound probe according to the first embodiment of the present invention.
FIG. 3 is a side view showing the ultrasound probe according to the first embodiment of the present invention.
FIG. 4 is a cross-sectional view showing an internal configuration of the ultrasound probe according to the first embodiment of the present invention.
FIG. 5 is a block diagram showing a configuration of an ultrasound diagnostic apparatus comprising the ultrasound probe according to the first embodiment of the present invention.
FIG. 6 is a block diagram showing an internal configuration of a transmission/reception circuit of the ultrasound probe according to the first embodiment of the present invention.
FIG. 7 is a block diagram showing an internal configuration of an image generation unit of the ultrasound probe according to the first embodiment of the present invention.
FIG. 8 is a side view showing an ultrasound probe according to a second embodiment of the present invention.
FIG. 9 is a block diagram showing a configuration of an ultrasound diagnostic apparatus comprising an ultrasound probe according to a third embodiment of the present invention.

### DESCRIPTION OF THE PREFERRED EMBODIMENTS

Hereinafter, embodiments of the present invention will be described with reference to the drawings.

The following configuration requirements are described based on representative embodiments of the present invention, but the present invention is not limited to the embodiment.

In the present specification, a numerical range represented by "to" means a range including numerical values described before and after "to", both ends inclusive, as lower limit and upper limit values.

In the present specification, "same" and "identical" include an error range which is generally allowed in the technical field.

### First Embodiment

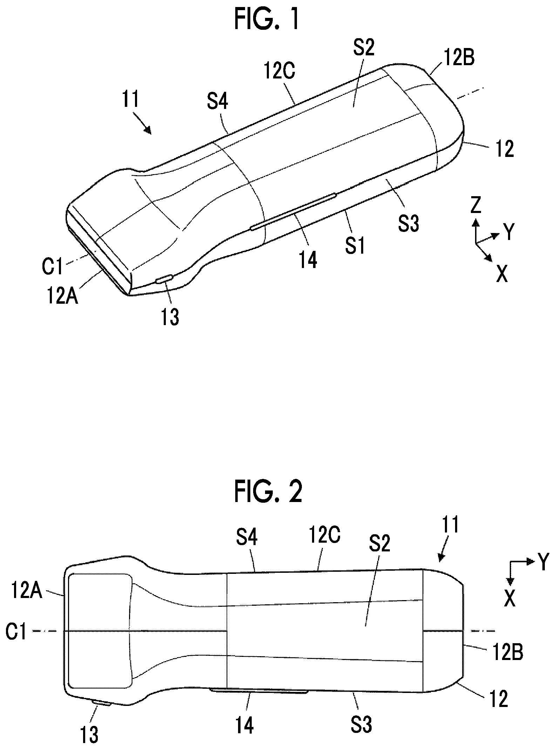

FIGS. 1 to 3 show an ultrasound probe 11 according to a first embodiment of the present invention. The ultrasound probe 11 comprises a housing 12, and the housing 12 extends in a predetermined direction as a whole and has a flat and wide shape. The housing 12 has a front end portion 12A disposed at one end portion in an extending direction, a rear end portion 12B disposed at the other end portion, and a grip portion 12C disposed between the front end portion 12A and the rear end portion 12B. The grip portion 12C is a portion gripped by a user in a case in which ultrasound diagnosis is performed using the ultrasound probe 11.

Here, for convenience, a direction from the front end portion 12A toward the rear end portion 12B will be referred to as a +Y direction, a width direction of the flat and wide housing 12 that is perpendicular to a Y direction will be referred to as an X direction, and a direction perpendicular to both the X direction and the Y direction will be referred to as a Z direction.

The housing 12 is made of, for example, an insulating resin, and the grip portion 12C has a tubular shape surrounded by four side plate portions extending along a center line C1 extending from the front end portion 12A to the rear end portion 12B. The four side plate portions are composed of a first side plate portion S1 directed in a -Z direction, a second side plate portion S2 directed in a +Z direction on a side opposite to the first side plate portion S1, a third side plate portion S3 connecting the first side plate portion S1 and the second side plate portion S2 and directed in a +X direction, and a fourth side plate portion S4 connecting the first side plate portion S1 and the second side plate portion S2 and directed in a -X direction.

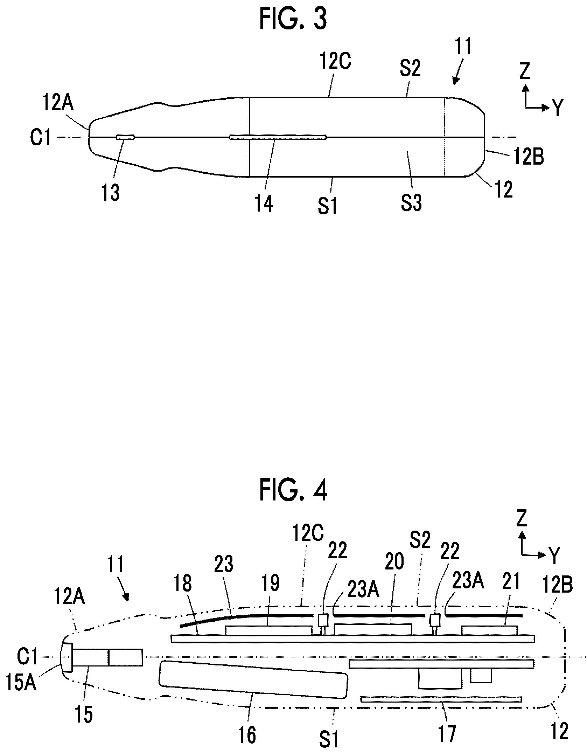

As shown in FIG. 3, the housing 12 has an outer shape in which a thickness in the Z direction gradually decreases from a vicinity of a center portion in the Y direction toward the front end portion 12A along the center line C1, although there is a slight protrusion portion in a case of being viewed in the X direction.

In addition, a protrusion 13 for indicating an orientation of the ultrasound probe 11 is formed to protrude on the +X direction side of the front end portion 12A, and a light-emitting unit 14 extending in the Y direction along the center line C1 is disposed on an outer surface of the third side plate portion S3.

FIG. 4 shows an internal configuration of the ultrasound probe 11.

The transducer array 15 is disposed inside the front end portion 12A of the ultrasound probe 11 at a position through which the center line C1 passes. The transducer array 15 includes a plurality of transducers arranged in the X direction, and an ultrasound emission surface 15A of the transducer array 15 is exposed from the housing 12 and directed in the -Y direction.

A flat plate-shaped battery 16 is disposed inside the housing 12 at a position offset toward the front end portion 12A side from the grip portion 12C so as to lie along an inner surface of the housing 12. The battery 16 is disposed to be offset toward the -Z direction side with respect to the center line C1.

As described above, since the housing 12 has the outer shape in which the thickness in the Z direction gradually decreases from the vicinity of the center portion in the Y direction toward the front end portion 12A along the center line C1, the battery 16 is disposed at a position offset toward the front end portion 12A side from the grip portion 12C so as to lie along the inner surface of the housing 12, so that the battery 16 is in a state of being inclined relative to the center line C1.

A power receive coil 17 is disposed on the +Y direction side of the battery 16 at a position offset toward the -Z direction side with respect to the center line C1. The power receive coil 17 has a thinner flat plate shape than the battery 16 and is disposed along an inner surface of the first side plate portion S1 of the grip portion 12C. Here, at least a region of the first side plate portion S1 in which the power receive coil 17 is disposed has a planar inner surface and a planar outer surface, and the power receive coil 17 is disposed in a state of being in contact with the inner surface of the first side plate portion S1 or being extremely close to the inner surface of the first side plate portion S1.

In addition, inside the housing 12, a circuit board 18 is disposed at a position offset toward a side opposite to the battery 16 and the power receive coil 17 with respect to the center line C1, that is, inside the second side plate portion S2 of the grip portion 12C. The circuit board 18 extends from the grip portion 12C to the vicinity of the transducer array 15 of the front end portion 12A along an XY plane, and two integrated circuits 19 and 20 and a wireless communication circuit 21 are sequentially mounted on a front surface of the circuit board 18 on the +Z direction side along the center line C1 in the +Y direction.

Furthermore, two temperature sensors 22 connected to the circuit board 18 are disposed inside the housing 12. Among the two temperature sensors 22, one temperature sensor 22 is located between the two integrated circuits 19 and 20, and the other temperature sensor 22 is located between the integrated circuit 20 and the wireless communication circuit 21, and each of the temperature sensors 22 is disposed close to an inner surface of the second side plate portion S2 of the grip portion 12C.

In addition, a sheet-like heat dissipation member 23 is disposed between the circuit board 18 and the inner surface of the second side plate portion S2 of the grip portion 12C, and the two integrated circuits 19 and 20 and the wireless communication circuit 21 mounted on the circuit board 18 are covered with the heat dissipation member 23. The heat dissipation member 23 consists of, for example, a resin sheet in which the thermal conductivity is improved by encapsulating a high thermal conductive filler, and efficiently absorbs heat from the integrated circuits 19 and 20 and the wireless communication circuit 21, which are heat generating bodies during operation, to prevent malfunction and failure of the integrated circuits 19 and 20 and the wireless communication circuit 21 from occurring in advance.

However, the heat dissipation member 23 has two opening portions 23A formed corresponding to positions at which the two temperature sensors 22 are disposed, and the two temperature sensors 22 face the inner surface of the second side plate portion S2 through the corresponding opening portions 23A of the heat dissipation member 23.

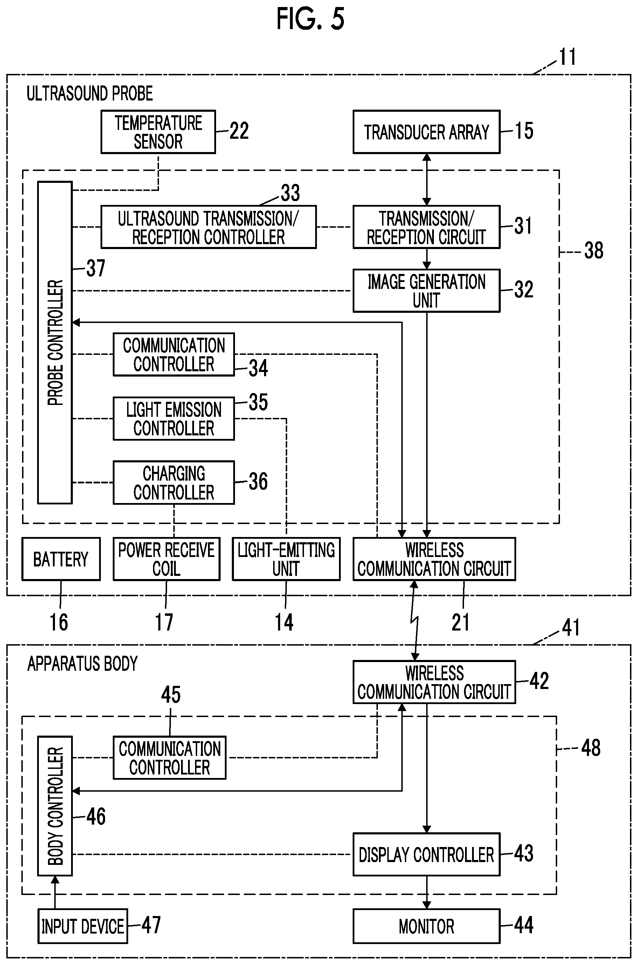

Hereinafter, FIG. 5 shows a configuration of an ultrasound diagnostic apparatus comprising the ultrasound probe 11 according to the first embodiment. The ultrasound diagnostic apparatus comprises the ultrasound probe 11 and an apparatus body 41 according to the first embodiment, and the ultrasound probe 11 and the apparatus body 41 are connected to each other by wireless communication.

The ultrasound probe 11 has a transmission/reception circuit 31 connected to the transducer array 15, and an image generation unit 32 and the wireless communication circuit 21 are sequentially connected to the transmission/reception circuit 31. In addition, an ultrasound transmission/reception controller 33 is connected to the transmission/reception circuit 31. Further, a communication controller 34 is connected to the wireless communication circuit 21, a light emission controller 35 is connected to the light-emitting unit 14, and a charging controller 36 is connected to the power receive coil 17. In addition, a probe controller 37 is connected to the temperature sensors 22, the ultrasound transmission/reception controller 33, the communication controller 34, the light emission controller 35, and the charging controller 36. Here, the wireless communication circuit 21 and the probe controller 37 are connected to each other so as to enable bidirectional exchange of information.

Further, a processor 38 on the ultrasound probe 11 side is formed by the transmission/reception circuit 31, the image generation unit 32, the ultrasound transmission/reception controller 33, the communication controller 34, the light emission controller 35, the charging controller 36, and the probe controller 37.

The apparatus body 41 comprises a wireless communication circuit 42, and a display controller 43 and a monitor 44 are sequentially connected to the wireless communication circuit 42. In addition, a communication controller 45 is connected to the wireless communication circuit 42, and a body controller 46 is connected to the wireless communication circuit 42, the display controller 43, and the communication controller 45. In addition, an input device 47 is connected to the body controller 46. Here, the wireless communication circuit 42 and the body controller 46 are connected to each other so as to enable bidirectional exchange of information.

Further, a processor 48 on the apparatus body 41 side is formed by the display controller 43, the communication controller 45, and the body controller 46.

In addition, the wireless communication circuit 21 of the ultrasound probe 11 and the wireless communication circuit 42 of the apparatus body 41 are connected to each other so as to enable bidirectional exchange of information, so that the ultrasound probe 11 and the apparatus body 41 are connected to each other by wireless communication.

The transducer array 15 of the ultrasound probe 11 includes a plurality of transducers arranged in a one-dimensional or two-dimensional manner. Each of these transducers transmits ultrasound waves in accordance with a drive signal supplied from the transmission/reception circuit 31, receives an ultrasound echo from a subject, and outputs a received signal. Each transducer is formed by, for example, forming electrodes on both ends of a piezoelectric body consisting of a piezoelectric single crystal represented by lead zirconate titanate (PZT), a polymeric piezoelectric element represented by poly vinylidene di fluoride (PVDF), or a piezoelectric single crystal represented by lead magnesium niobate-lead titanate (PMN-PT) solid solution.

The ultrasound transmission/reception controller 33 controls the transmission/reception circuit 31 to transmit an ultrasound beam and receive the ultrasound echo based on an instruction from the probe controller 37.

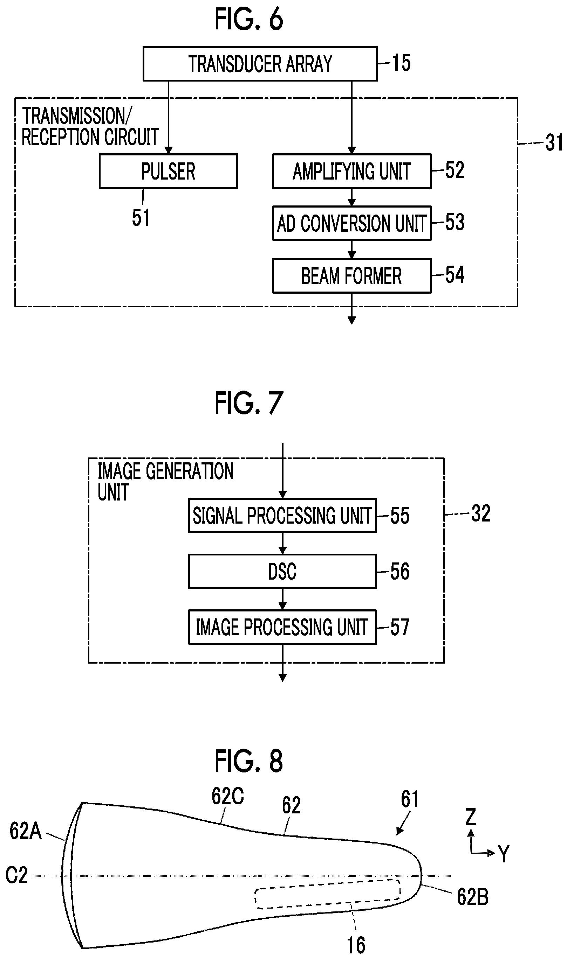

The transmission/reception circuit 31 transmits the ultrasound waves from the transducer array 15 and generates a sound ray signal based on the received signal acquired by the transducer array 15, under the control of the ultrasound transmission/reception controller 33. The transmission/reception circuit 31 includes, as shown in FIG. 6, a pulser 51 connected to the transducer array 15, and an amplifying unit 52, an analog-digital (AD) conversion unit 53, and a beam former 54 which are sequentially connected in series to the transducer array 15.

The pulser 51 includes, for example, a plurality of pulse generators, and supplies each of drive signals to the plurality of transducers by adjusting a delay amount such that the ultrasound waves transmitted from the plurality of transducers of the transducer array 15 form an ultrasound beam based on a transmission delay pattern selected in response to a control signal from the ultrasound transmission/reception controller 33. As described above, in a case in which a pulsed or continuous wave voltage is applied to the electrodes of the transducers of the transducer array 15, the piezoelectric body expands and contracts to generate a pulsed or continuous wave ultrasound wave from each transducer, and the ultrasound beam is formed from the combined wave of these ultrasound waves.

The transmitted ultrasound beam is reflected by a target, for example, a part of the subject, and an ultrasound echo propagates toward the transducer array 15 of the ultrasound probe 11. The ultrasound echo propagating toward the transducer array 15 in this manner is received by each of the transducers constituting the transducer array 15. In such a case, each transducer constituting the transducer array 15 expands and contracts by receiving the propagating ultrasound echo to generate the received signal that is an electric signal, and outputs the received signal to the amplifying unit 52.

The amplifying unit 52 amplifies the signal input from each of the transducers constituting the transducer array 15 and transmits the amplified signal to the AD conversion unit 53. The AD conversion unit 53 converts the signal transmitted from the amplifying unit 52 into digital reception data, and transmits the reception data to the beam former 54. The beam former 54 performs so-called reception focus processing by giving and adding delay with respect to each reception data converted by the AD conversion unit 53, in accordance with a sound velocity or a sound velocity distribution set based on a reception delay pattern selected in accordance with a control signal from the ultrasound transmission/reception controller 33. By the reception focus processing, each reception data, which is converted by the AD conversion unit 53, is phase-added, and the sound ray signal in which the focus of the ultrasound echo is narrowed down is generated. The sound ray signal generated in this way is sent to the image generation unit 32.

As shown in FIG. 7, the image generation unit 32 has a configuration in which a signal processing unit 55, a digital scan converter (DSC) 56, and an image processing unit 57 are sequentially connected in series.

The signal processing unit 55 performs correction of attenuation due to a distance in accordance with a depth of a reflection position of the ultrasound waves on the sound ray signal sent from the transmission/reception circuit 31, and then performs envelope detection processing to generate an image signal (B-mode image signal) which is tomographic image information related to a tissue in the subject.

The DSC 56 converts (raster-converts) the image signal generated by the signal processing unit 55 into an image signal in accordance with a normal television signal scanning method.

The image processing unit 57 performs various types of necessary image processing, such as brightness correction, gradation correction, sharpness correction, and color correction, on the image signal input from the DSC 56, to generate an ultrasound image signal. The ultrasound image signal generated by the image generation unit 32 in this way is sent to the wireless communication circuit 21.

The wireless communication circuit 21 includes an antenna for transmitting and receiving radio waves, and performs wireless communication with the wireless communication circuit 42 of the apparatus body 41. In this case, the wireless communication circuit 21 modulates a carrier based on the image signal sent from the image generation unit 32 to generate a transmission signal, and wirelessly transmits the generated transmission signal to the wireless communication circuit 42 of the apparatus body 41. As the carrier modulation method, for example, amplitude shift keying (ASK), phase shift keying (PSK), quadrature phase shift keying (QPSK), 16 quadrature amplitude modulation (16QAM), or the like is used.

The communication controller 34 controls the wireless communication circuit 21 such that the ultrasound image signal is transmitted with a transmission radio field intensity set by the probe controller 37.

The light emission controller 35 controls the light emission of the light-emitting unit 14 disposed on the outer surface of the third side plate portion S3 of the housing 12 such that various states of the ultrasound probe 11 are represented, under the control of the probe controller 37.

In a case in which the ultrasound probe 11 is disposed in a charger described later, the charging controller 36 controls charging of the battery 16 via the power receive coil 17 with respect to the battery 16 and the power receive coil 17 built in the housing 12.

The temperature sensors 22 disposed inside the housing 12 detect a temperature inside the housing 12, particularly, a temperature in the vicinity of the inner surface of the second side plate portion S2 of the grip portion 12C, and sends the detected temperature to the probe controller 37.

The battery 16 supplies the power to each unit in the ultrasound probe 11.

The probe controller 37 performs control of each unit in the ultrasound probe 11 based on a program or the like stored in advance.

The wireless communication circuit 42 of the apparatus body 41 includes an antenna for transmitting and receiving radio waves, and performs wireless communication with the wireless communication circuit 21 of the ultrasound probe 11. In this case, the wireless communication circuit 42 of the apparatus body 41 receives, for example, a transmission signal wirelessly transmitted from the wireless communication circuit 21 of the ultrasound probe 11 through the antenna, demodulates the received transmission signal, and outputs the ultrasound image signal. The wireless communication circuit 42 of the apparatus body 41 sends the ultrasound image signal output in this way to the display controller 43.

The display controller 43 performs predetermined processing on the ultrasound image signal sent from the wireless communication circuit 42 under the control of the body controller 46, and displays the ultrasound image on the monitor 44.

The monitor 44 displays the ultrasound image under the control of the display controller 43, and includes, for example, a display device such as a liquid crystal display (LCD) or an organic electroluminescence display (organic EL display).

The communication controller 45 controls the wireless communication circuit 42 of the apparatus body 41 such that the wireless communication circuit 42 receives the transmission signal from the wireless communication circuit 21 of the ultrasound probe 11.

The body controller 46 controls each unit of the apparatus body 41 based on a program stored in advance and an operation by the user via the input device 47.

The input device 47 is an input device for the user to perform an input operation, and is configured by, for example, a device such as a keyboard, a mouse, a trackball, a touchpad, and a touch sensor disposed in a state of being superimposed on the monitor 44.

Here, each of the processor 38 on the ultrasound probe 11 side including the transmission/reception circuit 31, the image generation unit 32, the ultrasound transmission/reception controller 33, the communication controller 34, the light emission controller 35, the charging controller 36, and the probe controller 37, and the processor 48 on the apparatus body 41 side including the display controller 43, the communication controller 45, and the body controller 46 may be configured by one or a plurality of types of hardware, and the type of the hardware is not limited. For example, the processor can be configured by hardware such as a central processing unit (CPU), a micro processing unit (MPU), a dedicated circuit such as an application specific integrated circuit (FPGA) for executing specific processing, a graphic processing unit (GPU), or a neural processing unit (NPU). Further, the processor includes each unit or each means that executes various types of processing in the present embodiment. Moreover, the type of hardware may be a combination of different types of hardware. In a case in which a plurality of types of hardware are configured to execute one or a plurality of types of processing of a certain processor, the plurality of types of hardware may exist in devices physically separated from each other or may exist in the same device. Further, in any embodiment, the order of each processing executed by the processor is not limited to the above-described order, and may be changed as appropriate. The hardware is configured by an electric circuit (circuitry) in which circuit elements, such as semiconductor elements, are combined, or the like.

In the first embodiment, the processor 38 on the ultrasound probe 11 side is configured by two integrated circuits 19 and 20 shown in FIG. 4.

Furthermore, the present embodiment may be implemented by hardware, software, firmware, microcode, or a combination thereof. Software, firmware, and microcode are implemented by programs. The program may be, for example, a program module group, and each function thereof may be implemented by a processor configured to execute each function. The program may be a program code or a plurality of code segments stored in one or a plurality of non-transitory computer-readable media (for example, a storage medium and other storages). The program may be stored in the plurality of non-transitory computer-readable media present in devices physically separated from each other. The program code or the code segment can represent any combination of procedures, functions, subprograms, routines, subroutines, modules, software packages, classes, instructions, data structures, or program statements. The program code or the code segment may be connected to another code segment or a hardware circuit by transmitting and receiving information, data, arguments, parameters, or contents in the memory.

In a case in which ultrasound diagnosis is performed by the ultrasound diagnostic apparatus shown in FIG. 5, first, under the control of the ultrasound transmission/reception controller 33 of the ultrasound probe 11, ultrasound beams are transmitted into the subject from the plurality of transducers of the transducer array 15 in accordance with the drive signal from the transmission/reception circuit 31. The ultrasound echo from the subject is received by the plurality of transducers of the transducer array 15, and the received signal, which is an analog signal, is output from the plurality of transducers to the transmission/reception circuit 31. The received signal is amplified by the amplifying unit 52 of the transmission/reception circuit 31, is subjected to AD conversion by the AD conversion unit 53, and is subjected to reception focus processing by the beam former 54, so that the sound ray signal is generated, and the sound ray signal is sent from the transmission/reception circuit 31 to the image generation unit 32.

Further, the image generation unit 32 generates the image signal, which is the tomographic image information related to the tissue in the subject, by performing correction of attenuation of the sound ray signal by the distance in accordance with the depth of the reflection position of the ultrasound waves and the envelope detection processing by the signal processing unit 55, the image signal is converted into the image signal in accordance with the normal television signal scanning method by the DSC 56, and the ultrasound image signal is generated by the image processing unit 57 further performing various types of necessary image processing such as gradation processing.

The ultrasound image signal generated in this way is wirelessly transmitted from the wireless communication circuit 21 of the ultrasound probe 11 to the apparatus body 41 and is received by the wireless communication circuit 42 of the apparatus body 41, and then the ultrasound image is displayed on the monitor 44 via the display controller 43.

As shown in FIG. 4, the housing 12 of the ultrasound probe 11 has an outer shape in which the thickness in the Z direction gradually decreases from the vicinity of the center portion in the Y direction toward the front end portion 12A along the center line C1, and the flat plate-shaped battery 16 is disposed at a position offset toward the front end portion 12A side from the grip portion 12C and disposed to be inclined relative to the center line C1 in the housing 12 so as to lie along the inner surface of the housing 12.

Here, in a case in which the flat plate-shaped battery 16 is to be disposed along the center line C1 without being inclined, there is a need to locate the battery 16 inside the tubular grip portion 12C instead of a position offset toward the front end portion 12A side from the grip portion 12C, and as a result, the battery 16 is moved to the +Y direction side as compared with a state shown in FIG. 4, and the power receive coil 17 is also moved to the +Y direction side in accordance with the movement of the battery 16, and thus the housing 12 is elongated along the center line C1.

That is, by disposing the battery 16 at a position offset toward the front end portion 12A side from the grip portion 12C and at an inclination relative to the center line C1, it is possible to reduce the size of the ultrasound probe 11 while accommodating a large number of components such as the transducer array 15, the battery 16, the power receive coil 17, the circuit board 18, the integrated circuits 19 and 20, the wireless communication circuit 21, and the temperature sensor 22 inside the housing 12.

Since at least a part of the outer surface of the second side plate portion S2 disposed on the side opposite to the first side plate portion S1 with the center line C1 interposed therebetween among the first side plate portion S1 to the fourth side plate portion S4 that surround the grip portion 12C has a curved shape, the ultrasound probe 11 in which the grip portion 12C of the housing 12 is easily gripped by the user is realized. Furthermore, since the housing 12 has the outer shape in which the thickness in the Z direction gradually decreases from the vicinity of the center portion in the Y direction toward the front end portion 12A in which the transducer array 15 is disposed along the center line C1, the user can easily perform a scan using the ultrasound emission surface 15A of the transducer array 15 along the body surface of the subject in a state of gripping the grip portion 12C with one hand, and the ultrasound probe 11 has excellent operability.

In addition, since the protrusion 13 is formed to protrude on the +X direction side of the front end portion 12A of the ultrasound probe 11, the user can easily grasp the orientation of the ultrasound probe 11 due to the presence of the protrusion 13 in a case in which the user grips the grip portion 12C, and the operability is improved.

Further, since the ultrasound probe 11 has the light-emitting unit 14 disposed on the outer surface of the third side plate portion S3 of the grip portion 12C, various states of the ultrasound probe 11 can be notified by changing a way of emitting light from the light-emitting unit 14 under the control of the light emission controller 35.

For example,
· Activation state of ultrasound probe 11,
· Wireless connection state between ultrasound probe 11 and apparatus body 41,
· Remaining capacity of battery 16,
· Charging state of battery 16,
· Error state, and
· Update state of software installed in ultrasound probe 11
are notified by changing the light emission color, the light emission pattern, and the like.

In addition, the temperature of the vicinity of the inner surface of the second side plate portion S2 of the grip portion 12C detected by the two temperature sensors 22 is transmitted to the probe controller 37, and the notification of a heat generation state of the ultrasound probe 11 can be performed by the light emitted from the light-emitting unit 14 under the control of the light emission controller 35.

In general, the surface temperature of the ultrasound probe is limited to a temperature equal to or lower than a temperature predetermined by a safety standard, but, in a case in which the way of the light emission of the light-emitting unit 14 is changed in accordance with the temperature detected by the temperature sensor 22, the user can easily and sensuously grasp the surface temperature of the ultrasound probe 11. In addition, the user may grasp a fact that the surface temperature of the ultrasound probe 11 exceeds a predetermined temperature based on the way of the light emission of the light-emitting unit 14, and may use the fact as a guide for temporarily suspending the use of the ultrasound probe 11.

In a case in which the temperature detected by the temperature sensors 22 has reached a predetermined threshold value, the surface temperature of the ultrasound probe 11 can be lowered by performing a treatment such as adjusting the drive signal supplied from the transmission/reception circuit 31 to the transducer array 15 by the ultrasound transmission/reception controller 33 to lower the frame rate of the ultrasound imaging or stopping the ultrasound imaging.

In the first embodiment described above, the protrusion 13 is formed to protrude on the +X direction side of the front end portion 12A of the ultrasound probe 11, but the present invention is not limited to this, and the protrusion 13 can also be formed to protrude on the -X direction side of the front end portion 12A of the ultrasound probe 11.

Similarly, in the first embodiment described above, the light-emitting unit 14 is disposed on the outer surface of the third side plate portion S3 of the grip portion 12C facing the +X direction, but the present invention is not limited to this, and the light-emitting unit 14 may be disposed on the outer surface of the fourth side plate portion S4 of the grip portion 12C facing the -X direction.

### Second Embodiment

In the ultrasound probe 11 according to the first embodiment, the housing 12 has the outer shape in which the thickness in the Z direction gradually decreases from the vicinity of the center portion in the Y direction toward the front end portion 12A along the center line C1, but the present invention is not limited to this.

FIG. 8 shows an ultrasound probe 61 according to a second embodiment.

The ultrasound probe 61 comprises a housing 62, and the housing 62 has a front end portion 62A that extends in the Y direction and that is disposed at a -Y direction end portion, a rear end portion 62B that is disposed at a +Y direction end portion, and a grip portion 62C that is disposed between the front end portion 62A and the rear end portion 62B. In addition, the housing 62 has an outer shape in which the thickness in the Z direction gradually decreases from the vicinity of the center portion in the Y direction toward the rear end portion 62B along a center line C2 extending from the front end portion 62A to the rear end portion 62B.

In addition, at least a part of the outer surface of the grip portion 62C has a curved shape.

A large number of components, such as the transducer array 15, the battery 16, the power receive coil 17, the circuit board 18, the integrated circuits 19 and 20, the wireless communication circuit 21, and the temperature sensor 22, are accommodated in the housing 62, as in the ultrasound probe 11 according to the first embodiment. However, the front end portion 62A of the housing 62 in which the transducer array 15 is disposed is formed to be thicker in the Z direction than the rear end portion 62B, and, for example, a two-dimensional transducer array, a linear transducer array, or the like having a relatively large ultrasound emission surface can be used as the transducer array 15.

In the housing 62, the flat plate-shaped battery 16 is disposed at a position offset toward the rear end portion 62B side from the grip portion 62C and disposed to be inclined relative to the center line C2 so as to lie along the inner surface of the housing 62.

Therefore, in the ultrasound probe 61 according to the second embodiment, it is possible to reduce the size of the ultrasound probe 61 while a large number of components are accommodated in the housing 62, similarly to the ultrasound probe 11 according to the first embodiment.

Since at least a part of the outer surface of the grip portion 62C has a curved shape, the user can easily grip the grip portion 62C of the housing 62, and the ultrasound probe 61 having excellent operability is realized.

### Third Embodiment

The ultrasound probe 11 according to the first embodiment includes the image generation unit 32, and the ultrasound image signal generated by the image generation unit 32 is wirelessly transmitted from the wireless communication circuit 21 of the ultrasound probe 11 to the apparatus body 41 as shown in FIG. 5, but the present invention is not limited to this.

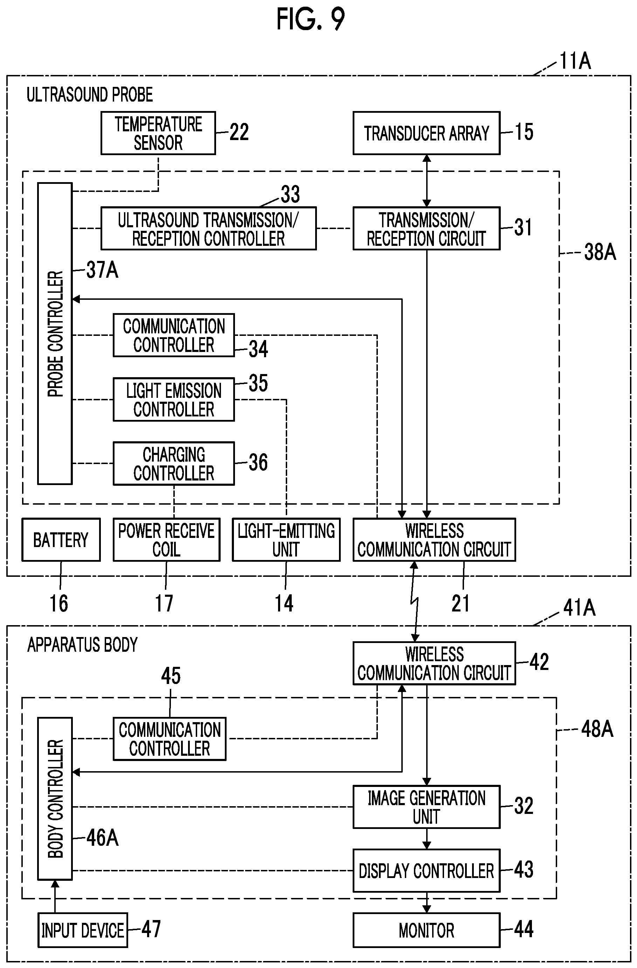

FIG. 9 shows a configuration of an ultrasound diagnostic apparatus comprising an ultrasound probe 11A according to a third embodiment. The ultrasound diagnostic apparatus comprises the ultrasound probe 11A and an apparatus body 41A according to the third embodiment, and the ultrasound probe 11A and the apparatus body 41A are connected to each other by wireless communication.

The ultrasound probe 11A is obtained by deleting the image generation unit 32 in the ultrasound probe 11 according to the first embodiment shown in FIG. 5, directly connecting the wireless communication circuit 21 to the transmission/reception circuit 31, and using a probe controller 37A instead of the probe controller 37, and other configurations of the ultrasound probe 11A are the same as those of the ultrasound probe 11 according to the first embodiment. In addition, the ultrasound probe 11A has the same housing 12 as the housing 12 in the ultrasound probe 11 according to the first embodiment.

The apparatus body 41A is obtained by newly connecting the image generation unit 32 between the wireless communication circuit 42 and the display controller 43 in the apparatus body 41 according to the first embodiment shown in FIG. 5, and connecting the body controller 46A to the display controller 43, the communication controller 45, and the image generation unit 32 instead of the body controller 46, and other configurations of the apparatus body 41A are the same as those of the apparatus body 41 according to the first embodiment.

In the ultrasound probe 11A, the transmission/reception circuit 31, the ultrasound transmission/reception controller 33, the communication controller 34, the light emission controller 35, the charging controller 36, and the probe controller 37A form a processor 38A on the ultrasound probe 11A side.

In addition, in the apparatus body 41A, the image generation unit 32, the display controller 43, the communication controller 45, and the body controller 46A form a processor 48A on the apparatus body 41A side.

The sound ray signal generated in the transmission/reception circuit 31 of the ultrasound probe 11A is wirelessly transmitted from the wireless communication circuit 21 to the apparatus body 41A, the image generation unit 32 performs attenuation correction and envelope detection processing on the sound ray signal received by the wireless communication circuit 42 of the apparatus body 41A to generate the ultrasound image signal, and the ultrasound image is displayed on the monitor 44 via the display controller 43.

In this way, in the ultrasound diagnostic apparatus comprising the ultrasound probe 11A according to the third embodiment as well, the ultrasound image can be displayed on the monitor 44 in the same manner as the ultrasound diagnostic apparatus comprising the ultrasound probe 11 according to the first embodiment.

In addition, the ultrasound probe 11A has the same housing 12 as the housing 12 in the ultrasound probe 11 according to the first embodiment, and the battery 16 is disposed at a position offset toward the front end portion 12A side from the grip portion 12C and disposed to be inclined relative to the center line C1 in the housing 12. Therefore, in the ultrasound probe 11A according to the third embodiment, it is possible to reduce the size of the ultrasound probe 11A while a large number of components are accommodated in the housing 12, similarly to the ultrasound probe 11 according to the first embodiment.

Furthermore, since at least a part of the outer surface of the grip portion 12C of the housing 12 has a curved shape, the grip portion 12C of the housing 12 is easily gripped by the user, and the ultrasound probe 11A having excellent operability is realized.

In the ultrasound probe 61 according to the second embodiment, similarly to the ultrasound probe 11A according to the third embodiment, a configuration can be adopted in which the image generation unit 32 is deleted, the wireless communication circuit 21 is directly connected to the transmission/reception circuit 31, and the apparatus body wirelessly connected to the ultrasound probe 61 includes the image generation unit 32.

As the apparatus body 41 used in the first embodiment and the apparatus body 41A used in the third embodiment, for example, a portable thin computer commonly referred to as a tablet or a stationary computer can be used.

### Explanation of References

11, 11A, 61: ultrasound probe
12, 62: housing
12A, 62A: front end portion
12B, 62B: rear end portion
12C, 62C: grip portion
13: protrusion
14: light-emitting unit
15: transducer array
15A: ultrasound emission surface
16: battery
17: power receive coil
18: circuit board
19, 20: integrated circuit
21, 42: wireless communication circuit
22: temperature sensor
23: heat dissipation member
23A: opening portion
31: transmission/reception circuit
32: image generation unit
33: ultrasound transmission/reception controller
34, 45: communication controller
35: light emission controller
36: charging controller
37, 37A: probe controller
38, 38A, 48, 48A: processor
41, 41A: apparatus body
43: display controller
44: monitor
46, 46A: body controller
47: input device
51: pulser
52: amplifying unit
53: AD conversion unit
54: beam former
55: signal processing unit
56: DSC
57: image processing unit
C1, C2: center line
S1: first side plate portion
S2: second side plate portion
S3: third side plate portion
S4: fourth side plate portion

## Claims

1. An ultrasound probe with a built-in battery, the ultrasound probe comprising:
a housing that has a front end portion and a rear end portion;
a transducer array that is disposed inside the front end portion of the housing; and
a battery that has a flat plate shape and that is disposed inside the housing,
wherein the housing has a center line extending from the front end portion to the rear end portion,
the housing has an outer shape that gradually tapers along the center line from a vicinity of a center portion of the center line toward the front end portion or the rear end portion, and
the battery is disposed inclined relative to the center line so as to lie along an inner surface of the housing.

2. The ultrasound probe according to claim 1,
wherein the battery is disposed at a position offset with respect to the center line.

3. The ultrasound probe according to either claim 1 or claim 2,
wherein the housing has an outer shape that gradually tapers from the vicinity of the center portion of the center line toward the front end portion, and
the battery is inclined relative to the center line at a position offset toward a side of the front end portion inside the housing.

4. The ultrasound probe according to any one of claims 1-3, further comprising:
a power receive coil that is disposed inside the housing,
wherein the transducer array, the battery, and the power receive coil are disposed in this order from the front end portion of the housing toward the rear end portion of the housing.

5. The ultrasound probe according to claim 4,
wherein the power receive coil has a flat plate shape.

6. The ultrasound probe according to either claim 4 or claim 5,
wherein the center line passes through the transducer array, and
the power receive coil is disposed parallel to the center line at a position offset from the center line.

7. The ultrasound probe according to any one of claims 4-6,
wherein the power receive coil is disposed at a position offset toward a side same as the battery with respect to the center line.

8. The ultrasound probe according to any one of claims 4-7, further comprising:
a circuit board that is disposed inside the housing at a position offset with respect to the center line toward a side opposite to the battery and the power receive coil; and
an integrated circuit that is mounted on the circuit board.

9. The ultrasound probe according to claim 8, further comprising:
a wireless communication circuit that is mounted on the circuit board.

10. The ultrasound probe according to any one of claims 1-9,
wherein the housing has an outer shape that gradually tapers from the vicinity of the center portion of the center line toward the rear end portion, and
the battery is inclined relative to the center line at a position offset toward a side of the rear end portion inside the housing.
